(19) Europäisches Patentamt

European Patent Office

Office européen des brevets

(11) **EP 1 162 940 B1**

(12) **EUROPÄISCHE PATENTSCHRIFT**

(45) Veröffentlichungstag und Bekanntmachung des Hinweises auf die Patenterteilung:
**23.11.2005 Patentblatt 2005/47**

(21) Anmeldenummer: **00920482.7**

(22) Anmeldetag: **09.03.2000**

(51) Int Cl.$^7$: **A61K 7/16**

(86) Internationale Anmeldenummer:
**PCT/EP2000/002039**

(87) Internationale Veröffentlichungsnummer:
**WO 2000/053154 (14.09.2000 Gazette 2000/37)**

(54) **SUBGINGIVALES PULVERSTRAHLEN**

SUBGINGIVAL TREATMENT BY POWDER JET

POUDRES SOUS FORME DE JET POUR TRAITEMENT SOUS-GINGIVAL

(84) Benannte Vertragsstaaten:
**AT BE CH CY DE DK ES FI FR GB GR IE IT LI LU MC NL PT SE**

(30) Priorität: **10.03.1999 DE 19910559**

(43) Veröffentlichungstag der Anmeldung:
**19.12.2001 Patentblatt 2001/51**

(73) Patentinhaber: **3M ESPE AG**
**82229 Seefeld (DE)**

(72) Erfinder:
• **FLEMMIG, Thomas**
**D-48161 Münster (DE)**

• **GANGNUS, Bernd**
**D-82346 Andechs (DE)**
• **GASSER, Oswald**
**D-82229 Seefeld (DE)**
• **GUGGENBERGER, Rainer**
**D-82211 Herrsching (DE)**

(56) Entgegenhaltungen:
**GB-A- 988 513**

• **PATENT ABSTRACTS OF JAPAN vol. 016, no. 081, 27. Februar 1992 (1992-02-27) & JP 03 271215 A (KAO), 3. Dezember 1991 (1991-12-03)**

**Beschreibung**

Die vorliegende Erfindung betrifft die Verwendung von feinkörnigen Pulvern in einem Verfahren zur Reinigung von Zahnwurzeloberflächen.

Etwa jeder fünfte bis siebte Erwachsene leidet an einer marginalen Parodontitis. Parodontalerkrankungen werden durch Bakterien verursacht, die sich an den Zahn anlagern, dort einen als bakterielle Plaque bezeichneten Biofilm bilden und die Zahnfleischtaschen besiedeln. Die Erkrankung ist durch eine Entzündung der parodontalen Weichgewebe, Freiliegen der Zahnwurzeloberfläche, Bildung parodontaler Taschen sowie progredienten Abbau des zahntragenden Faserapparates und des Alveolarknochens gekennzeichnet. Unbehandelt führen marginale Parodontitiden nicht selten zum Zahnverlust. Zahnstein, der sich durch Mineralisation der abgestorbenen bakteriellen Plaque bildet, ist selbst nicht pathogen. Er ist aber in der Regel mit einer lebenden bakteriellen Plaque bedeckt.

Ein wesentliches Ziel der Parodontitistherapie ist, die progrediente Zerstörung des Zahnhalteapparates aufzuhalten und somit dem Zahnverlust durch Parodontalerkrankung vorzubeugen. Hierzu werden die bakterielle Plaque und der Zahnstein von allen Zahnoberflächen oberhalb (supragingival) und soweit zugängig auch unterhalb des Zahnfleischsaums (subgingival) entfernt. Bei fortgeschrittenen Parodontalerkrankungen sind häufig zusätzlich parodontalchirurgische Eingriffe notwendig, um die subgingival gelegenen Zahnwurzelareale vollständig zu reinigen. Diese Behandlung führt zu einer nur kurzfristigen Heilung der parodontalen Gewebe. Da es auch bei adäquater Mundhygiene innerhalb weniger Monate zur fast vollständigen bakteriellen Rekolonisation der parodontalen Tasche kommt (Haffajee et al. 1997, J Clin Periodontol 24:324-334), muß zur Gesunderhaltung des marginalen Parodontiums die neu gebildete Plaque in drei- bis sechsmonatigen Abständen professionell entfernt werden (Axelsson und Lindhe 1981, J Clin Periodontol 8:281-294). Bei dieser unterstützenden Parodontitistherapie kommen hauptsächlich Küretten, Schall- oder Ultraschallscaler zum Einsatz. Die Anwendung dieser Instrumente ist für den Behandler technisch anspruchsvoll und wird von den Patienten meist als unangenehm empfunden. Bei wiederholter Reinigung im Rahmen der unterstützenden Parodontitistherapie kommt es kumulativ zu einem klinisch relevanten Wurzelabtrag, der zur Hypersensibilität und Schwächung der Wurzel bis hin zur Perforation des Wurzelkanalsystems und Frakturgefährdung führen kann (Zappa et al. 1991, J Periodontol 62:750-754, Flemmig et al. 1998, J Periodontol 69:547-533).

Supragingivale Zahnflächen lassen sich sehr effizient mit einem Pulver-Luft-Wasser-(PLW)-Strahl reinigen (US-A-45- 953 65). Das bei PLW-Strahlgeräten bisher verwendete Strahlmittel (Natriumhydrogencarbonat) ist in bezug auf dessen Abrasivität für die Reinigung des Zahnschmelzes unkritisch, jedoch führt es bei Anwendung auf der Wurzel schon in kurzer Zeit zu klinisch relevantem Substanzabtrag (Boyde 1984, Brit Dent J 156:287-291). Da bei marginalen Parodontitiden die Zahnwurzel freiliegt, hat der PLW-Strahl unter Verwendung der bisher üblichen Strahlmittel bei der unterstützenden Parodontitistherapie einen eingeschränkten Einsatz.

In der GB 1 480 594 wird ein Verfahren zur Zahnreinigung unter Verwendung eines Wasserstrahls offenbart. Der Wasserstrahl enthält dabei Teilchen einer Härte, die die Zerstörung der Zahnhartsubstanz (Enamel) vermeidet. Beispielhaft werden Calciumlactat, kristalline Fruchtsäuren, Dextrine, Gelatine, kristalline schwache Säuren, Mineralsalze und Knochenleim genannt. Nachteilig an diesem Verfahren ist, daß die offenbarten Substanzen sich nicht in jedem Fall zur Reinigung subgingivaler Zahnhartsubstanz eignen.

Aufgabe der vorliegenden Erfindung ist es daher, einfache und schonende Mittel und Wege für die Reinigung von Zahnwurzeloberflächen, d.h., subgingivaler Zahnhartsubstanz zur Verfügung zu stellen.

Gelöst wird diese Aufgabe durch die Bereitstellung und Verwendung von Pulvern bzw. Pulvergemischen gemäß den Ansprüchen.

Diese Pulver besitzen vorzugsweise eine Abrasivität, die zu einem um mindestens 50% geringeren Abtrag an Wurzeldentin führen als Pulver bzw. Pulvergemische auf Basis von beispielsweise Natriumhydrogencarbonat der mittleren Korngröße von ca. 55 μm, welche üblicherweise zur Reinigung von supragingivalen Zahnflächen eingesetzt werden.

Es wurde überraschenderweise gefunden, daß sich Pulver bzw. Pulvergemische, welche diese Bedingung erfüllen, in hervorragender Weise zur Reinigung von Wurzeldentin eignen, ohnc dabei merkliche Mengen an gesunder Zahnwurzelsubstanz abzutragen.

Der Behandler geht mit Hilfe der hier offenbarten Pulver bzw. Pulvergemischen so vor, daß er den Pulverstrahl auf die zu reinigende Stelle richtet und durch kurzes Abstrahlen der Wurzeldentinoberfläche die Reinigung durchführt. Da der Pulverstrahl dabei auch einige mm tief in die parodontale Tasche einpenetriert, ist es oftmals nicht notwendig, wie bei der herkömmlichen Methode, zuvor das Zahnfleisch operativ zu eröffnen. Für den Behandler bedeutet dies eine erhebliche Zeitersparnis, für den Patienten eine deutlich geringere Belastung, da sogar auf eine Lokalanästhesie verzichtet werden kann.

Im folgenden wird die Erfindung näher erläutert.

Pulver bzw. Pulvergemische, die sich gemäß der Ansprüche für die Verwendung im Rahmen der vorliegenden Erfindung eignen, sind solche, die sich mittels herkömmlicher Pulverstrahlgeräte für den Dentalbereich fördern lassen.

Die Pulver bzw. Pulvergemische weisen eine mittlere Korngröße von nicht mehr als 45 μm auf.

Die Korngröße der Pulver bzw. Pulvergemische liegt dabei in einem Bereich von 0,01 µm bis 200 µm. Bevorzugt ist eine Korngröße im Bereich von 0,05 µm bis 60 µm, insbesondere von 0,1 bis 45 µm. Die mittlere Korngröße ist üblicherweise kleiner 45 µm, vorzugsweise kleiner 35 µm.

Die Dicht des Pulver bzw. Pulvergemisches beträgt nicht merh als $2,09/cm^3$

Ein entscheidendes Kriterium für die Verwendbarkeit der Pulver bzw. Pulvergemische ist deren Abrasivität gegenüber Wurzeldentin, d.h. subgingivaler Zahnhartsubstanz. Der Zeitbedarf für die Reinigung einer Zahnwurzeloberfläche im Rahmen einer Parodontalbehandlung unter Verwendung der hier offenbarten Pulver bzw. Pulvergemische beträgt ca. eine Minute. Dies bedeutet, daß innerhalb dieser Zeit kein wesentlicher Abtrag von Wurzeldentin erfolgen sollte.

Zur Auswahl geeigneter Pulver bzw. Pulvergemische wurde deshalb ein Test entwickelt bei dem unter Laborbedingungen die Abrasivität von Strahlmitteln gegenüber Wurzeldentin ermittelt werden kann. Die Abrasivität der besagten Pulver bzw. Pulvergernische wird dabei so ermittelt, daß eine Rinderwurzeldentinfläche von $9,6\ mm^2$ mit einem Aufbringdruck von 4,0 bar aus einem Abstand von 2,3 mm 1 Minute lang bestrahlt wird. Anschließend wird die bearbeitete Oberfläche unter dem Mikroskop vermessen und aus diesen Messdaten das Volumen an abgetragenem Rinderdentin berechnet. Die zur Reinigung von Zahnschmelz üblicherweise eingesetzten kommerziell erhältlichen Pulver (z.B. Air-Flow-Pulver, Fa. EMS) zeigen in diesem Test einen Abtrag von ca. $1,24\ mm^3$, der auch optisch deutlich zu erkennen und klinisch nicht akzeptabel ist.

Überraschend wurde nun gefunden, daß bestimmte Pulver bzw. Pulvergemische bei der oben beschriebenen Vorgehensweise eine deutlich geringere Abrasivität aufweisen, die aber dennoch hoch genug ist, um unerwünschte Beläge auf dem Wurzeldentin zu entfernen und somit eine effiziente Pulverstrahlreinigung von Zahwurzeloberflächen ermöglichen.

Als für zur Reinigung von Wurzeloberflächen gut geeignete Pulver haben sich beispielsweise Aminosäuren, Zucker, organische Säuren und deren Salze, insbesondere Glycin, Harnstoff, Kaliumhydrogenphthalat oder Kalium-D-Glukonat gezeigt. Die Pulver werden dabei bevorzugt mit einer Korngrößenverteilung von 0,05 µm bis 60 µm, besonders bevorzugt mit einer Korngrößenverteilung von 0,1 µm bis 45 µm eingesetzt.

Selbstverständlich sind auch Pulvergemische aus mindestens zwei Pulvern für den beschriebenen Zweck geeignet. Das Mischungsverhältnis ist dabei grundsätzlich beliebig, liegt aber bei Verwendung zweier Pulver vorzugsweise im Bereich von 1 : 10 bis 10 : 1 bezogen auf die Masse der zu mischenden Pulver.

Allen beispielhaft genannten Pulvern gemeinsam ist, daß sie üblicherweise eine geringere Dichte aufweisen als bislang eingesetzte Pulver bzw. Pulvergemische, die für die supragingivale Zahnreinigung verwendet werden.

In Versuchen hat sich nun überraschenderweise herausgestellt, daß diese bislang bereits für die supragingivale Anwendung eingesetzten Pulver erst dann für die Reinigung von Wurzeloberflächen geeignet sind, wenn sie deutlich feiner gemahlen sind und damit geringere mittlere Korngrößen aufweisen.

Es kann vorteilhaft sein, die zuvor genannten Pulver mit weiteren, sehr feinteilig vorliegenden Pulvern zu vermischen, bevor sie als Reinigungsmittel für Zahnwurzeloberflächen verwendet werden. Dies kann bewirken, daß sich die dabei entstehenden Pulvermischungen mit herkömmlichen Pulverstrahlgeräten besser und schneller fördern lassen.

Als solche sehr feinteilig vorliegende Pulver seien beispielhaft hochdisperse Kieselsäuren oder Aerosile genannt, vorzugsweise mit einer durchschnittlichen Korngröße von ca. 0,07 µm. Die zusetzbare Menge liegt vorzugsweise im Bereich von 0,001 bis 5,0 Gew.-%, besonders bevorzugt im Bereich von 0,01 bis 0,1 Gew-% bezogen auf die Gesamtmasse des Pulvers.

Denkbar ist auch die Zumischung von anderen feinteiligen Substanzen, beispielsweise von Bleichmitteln, wie Perborate (z.B. Natriumperborat), fluoridfreisetzenden Substanzen wie Natriumfluorid, Analgetika wie Articain oder Lidocain, Bakterioziden wie Chlorhexidin oder Triclosan, Geschmacksstoffen wie Zitronensäure und/oder Ascorbinsäure. Die zusetzbare Menge liegt vorzugsweise im Bereich von 0,001 bis 5,0 Gew.%, besonders bevorzugt im Bereich von 0,01 bis 0,1 Gew-% bezogen auf die Gesamtmasse des Pulvers.

Je nach zugemischter Substanz kann der Anwendungsbereich der beschriebenen Pulver erweitert werden. Die Verwendung von Geschmacksstoffen kann beispielsweise die Patientenakzeptanz erhöhen.

Die Pulver können gegebenenfalls auch oberflächenbeschichtet sein. Als geeignete Oberflächenbeschichtungsmittel seien genannt: Stärke, Alginate, Collagen (Gelatine), Hydrogele, Polyanhydride, Polyester, Polyiminocarbonate, Polycaprolactone, Polyaminosäuren, Polyphosphazene. Hierbei hat sich die Anwendung der Mikrokapseltechnologie als vorteilhaft herausgestellt.

Über kontrollierte Kristallisation kann darüberhinaus auch die Oberflächenstruktur beeinflußt werden, um eine gewünschte Abrasivität zu erreichen. Als günstig haben sich die Kristallisationsformen monoklin, rhomboide Prismen, tetragonal-scalenohedral und orthorhombisch erwiesen.

*Vorbereitung der Rinderzahnwurzeln und Durchführung der Messungen:*

Pro Versuch wurden je 3 frisch extrahierte Rinderzähne verwendet, deren Wurzelbereich nach Reinigung durch Abspülen mit entionisiertem Wasser oberflächlich durch Behandlung mit Schleifpapier geglättet wurde. Die so vorbereiteten Rinderzahawurzeln wurden in einer Einbettmasse (Reprogum®, Fa. Espe, Seefeld) fixiert und mit einer Kunststoffplatte abgedeckt, welche eine kreisrunde Aussparung mit einem Durchmesser von 3,5 mm aufwies. Die freiliegende Rinderwurzeldentinfläche wurde anschließend für eine Minute mittels eines Pulverstrahlgerätes (Airflow®, Fa. EMS, München) mit dem entsprechenden Pulver bzw. Pulvergemisch bei einem Strahldruck von 4,0 bar und einem Abstand zwischen Wurzeloberfläche zu Strahldüse von 2,3 mm bestrahlt. Für jeden Versuch wurden jeweils maximal befüllte Pulvertanks verwendet.

Zur Ermittlung des abgetragenen Rinderwurzeldentinvolumens wurden die abgestrahlten Oberflächen mittels einer Abformmasse (Dimension Garant®, Fa. Espe, Seefeld) dubliert. Das dabei in Form eines Halbelipsoids entstandene Negativ des abgetragenen Volumens wurde unter einem Lichtmikroskop entlang seiner Achsen vermessen und anhand dieser Daten mit Hilfe folgender Formel der Volumenabtrag berechnet:

$$\text{Abgetragenes Volumen} = \frac{2}{3}\,\pi\,a \bullet b \bullet c$$

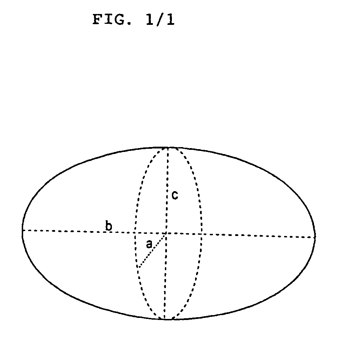

**Abbildung 1: Darstellung des Halbelipsoids zur Berechnung des Volumenabtrages.**

*Beispiel I: Herstellungeiner erfindungsgem äßen Pulvermischung I*

100 g Glycin (Fa.Fluka, Deisenhofen) wurden für 3 Minuten in einer Achatscheibenmühle gemahlen und anschließend trocken über ein 40 µm Sieb gesiebt. Anschließend wurde das so gewonnene Pulver mit 0,36 g HDK-H-2000 (Fa. Degussa, Hanau) versetzt und diese Mischung nochmals über ein 60 µm Sieb gesiebt.

*Beispiel II. Herstellung einer erfindungsgemäßen Pulvermischung II*

100 g Kalium-D-Glukonat (Fa.Fluka, Deisenhofen) wurden für 4 Minuten in einer Achatscheibenmühle gemahlen und anschließend trocken über ein 40 µm Sieb gesiebt. Anschließend wurde das so gewonnene Pulver mit 0,63 g HDK-H-2000 (Fa. Degussa, Hanau) versetzt und diese Mischung nochmals über ein 60 µm Sieb gesiebt.

*Beispiel III: Herstellung einer erfndungsgemäßen Pulvermischung III*

100 g Kaliumhydrogenphthalat (Fa.Fluka, Deisenhofen) wurden für 3 Minuten in einer Achatscheibenmühle gemahlen und anschließend trocken über ein 40 µm Sieb gesiebt. Anschließend wurde das so gewonnene Pulver mit 0,79 g HDK-H-2000 (Fa. Degussa, Hanau) versetzt und diese Mischung nochmals über ein 60 µm Sieb gesiebt.

*Beispiel IV: Herstellung einer erfindungsgemäßen Pulvermischung IV*

100 g Harnstoff (Fa.Fluka, Deisenhofen) wurden für 2 Minuten in einer Achatscheibenmühle gemahlen und anschließend trocken über ein 40 µm Sieb gesiebt. Anschließend wurde das so gewonnene Pulver mit 0,18 g HDK-H-2000 (Fa. Degussa, Hanau) versetzt und diese Mischung nochmals über ein 60 µm Sieb gesiebt.

*Beispiel V: Herstellung einer erfindungsgemäßen Pulvermischung V*

100 g Natriumhydrogencarbonat (Fa.Fluka, Deisenhofen) wurden für 2,5 Minuten in einer Achatscheibenmühle gemahlen und anschließend trocken über ein 40 µm Sieb gesiebt. Anschließend wurde das so gewonnene Pulver mit 0,19 g HDK-H-2000 (Fa. Degussa, Hanau) versetzt und diese Mischung nochmals über ein 60 µm Sieb gesiebt.

*Beispiel VI: Herstellung einer erfindungsgemäßen Pulvermischung VI*

100 g Natriumascorbat (Fa.Fluka, Deisenhofen) wurden für 1 Minute in einer Achatscheibenmühle gemahlen und anschließend trocken über ein 40 µm Sieb gesiebt. Anschließend wurde das so gewonnene Pulver mit 0,9 g HDK-H-2000 (Fa. Degussa, Hanau) versetzt und diese Mischung nochmals über ein 60 µm Sieb gesiebt.

*Beispiel VII: Herstellung einer erfindungsgemäßen Pulvermischung VII:*

100 g Air-Flow-Pulver (Fa. EMS) wurden für 1 Minute in einer Achatscheibenmühle gemahlen und anschließend trocken über ein 40 µm Sieb gesiebt. Anschließend wurde das so gewonnene Pulver mit 0,9 g HDK-H-2000 (Fa. Degussa, Hanau) versetzt und diese Mischung nochmals über ein 60 µm Sieb gesiebt.

*Referenzbeispiel: nicht erfindungsgemäß*

100g Air-Flow-Pulver (Fa. EMS) wurden wie vom Hersteller geliefert eingesetzt.

Die so gewonnene Pulvermischungen I-VII, sowie das Referenzpulver wurden in ein Pulverstrahlgerät (Airflow®, Fa. EMS, München) gefüllt und wie oben beschrieben verwendet. Die jeweilige Menge an abgetragenem Rinderwurzeldentin ist Tabelle 1 zu entnehmen.

Tabelle 1:

| Abgetragenes Volumen an Rinderwurzeldentin in Abhängigkeit der verwendeten Pulvermischung bzw. deren Dichte und mittleren Korngröße. | | | |
|---|---|---|---|
| Pulvermischung | Dichte [g/cm$^3$]* | Mittlere Korngröße [µm]** | Abgetragenes Volumen [mm$^3$] |
| I | 1,16 | 10,7 | 0,07 |
| II | 1,73 | 21,7 | 0.043 |
| III | 1,64 | 10,7 | 0,062 |
| IV | 1,34 | Ca. 12 (geschätzt) | 0,022 |
| V | 2,16 | 35,9 | 0,441 |
| VI | 1,80 | 21,0 | 0,206 |
| VII | 2,16 | 34,8 | 0,440 |
| Referenz | 2,16 | 54,3 | 1,24 |

\* Quelle: Beilstein

\*\* Gemessen an Granulometer der Fa. CILAS mit Isopropanol als Dispergiermittel

Die Dichte der verwendbaren Pulver entspricht dabei den in herkömmlichen Nachschlagewerken angegebenen Werten und ist abhängig von der jeweiligen Kristallstruktur. Die Korngrößenverteilung und die mittlere Korngröße läßt sich über dem Fachmann bekannte Methoden wie Siebverfahren oder Granulometer (z.B. Fa. Silas) bestimmen.

**Patentansprüche**

1.   Verwendung feinkörniger.Pulver bzw. Pulvergemische zur Herstellung eines Mittels für die Pulverstrahlreinigung von subgingivaler Zahnhartsubstanz, wobei die Pulver eine mittlere Korngröße von nicht mehr als 45 µm und eine Dichte von nicht mehr als 2,0 g/cm$^3$ aufweisen.

2. Verwendung nach Anspruch 1, wobei die Pulver oder Pulvergemische einen weiteren feinteiligen Stoff umfassen.

3. Verwendung nach Anspruch 2, wobei der feinteilige Stoff gewählt ist aus Kieselgel, Bleichmitteln, Analgetika, Bakterioziden und/oder Geschmacksstoffen.

4. Verwendung nach einem der vorstehenden Ansprüche, wobei als bevorzugte Pulver bzw. Pulvergemische Aminosäuren, Zucker und/oder organische Säuren sowie deren Salze insbesondere die Alkali-, Erdalkali- und/oder Ammoniumsalze eingesetzt werden.

5. Verwendung nach einem der vorstehenden Ansprüche, wobei die Pulver bzw. Pulvergemische mittels eines Strahldruckgerätes ausgebracht werden.

**Claims**

1. Use of finely particulate powders or powder mixtures for producing a composition for the powder jet cleaning of subgingival hard dental tissue, the powders having an average particle size of not more than 45 μm and a density of not more than 2.0 g/cm$^3$.

2. Use according to Claim 1, the powders or powder mixtures comprising a further finely divided substance.

3. Use according to Claim 2, the finely divided substance being chosen from silica gel, bleaching agents, analgesics, bactericides and/or flavourings.

4. Use according to one of the above claims, the preferred powders or powder mixtures employed being amino acids, sugars and/or organic acids and their salts, in particular the alkali metal, alkaline earth metal and/or ammonium salts.

5. Use according to one of the above claims, the powders or powder mixtures being applied by means of a pressure jet apparatus.

**Revendications**

1. Utilisation de poudres ou de mélanges de poudres à grains fins pour la fabrication d'un agent pour le nettoyage par jet de poudre, d'une substance dentaire dure sub-gingivale, dans laquelle les poudres présentent une taille granulaire moyenne de 45 μm au plus et un poids volumique de 2,0 g/cm$^3$ au plus.

2. Utilisation selon la revendication 1, dans laquelle les poudres ou les mélanges de poudres comprennent un autre matériau finement divisé.

3. Utilisation selon la revendication 2, dans laquelle le matériau finement divisé est choisi parmi du gel de silice, des agents de blanchiment, des antalgiques, des bactéricides et/ou des agents sapides.

4. Utilisation selon l'une quelconque des revendications précédentes, dans laquelle sont utilisés comme poudres ou mélanges de poudres préférés, des acides aminés, des sucres et/ou des acides organiques ainsi que leurs sels, en particulier, leurs sels de métaux alcalins, de métaux alcalinoterreux et/ou d'ammonium.

5. Utilisation selon l'une quelconque des revendications précédentes, dans laquelle les poudres ou les mélanges de poudres sont appliqués au moyen d'un appareil à jet sous pression.

FIG. 1/1